# EUROPÄISCHE PATENTANMELDUNG

(11) **EP 0 724 152 A2**
(43) Veröffentlichungstag der Anmeldung: **31.07.1996**
(21) Anmeldenummer: 96100957.8
(22) Anmeldetag: 24.01.1996
(51) Int. Cl.: G01N 27/416, G01N 27/49, G01N 33/483

(54) **Vorrichtung, Verfahren und Messelektrode zum Bestimmen von Flüssigkeitsparametern**

(30) Priorität: 25.01.1995 PL 30696095; 19.09.1995 PL 31054395
(71) Anmelder: PRZEDSIEBIORSTWO ZAGRANICZNE HTL, PL-03-230 Warszawa (PL)
(72) Erfinder: Czernecki, Andrzej, 03-934 Warszawa (PL); Bylina, Andrzej, 05-120 Legionowo (PL); Kawiak, Jan, 01-204 Warszawa (PL); Ohnsorge, Piotr, 01-493 Warszawa (PL)
(74) Vertreter: Hennicke, Albrecht, Dipl.-Ing.

(57) **Zusammenfassung**

Behälter zur Aufnahme von Kalibrierflüssigkeiten und/oder anderen Betriebsflüssigkeiten für eine Vorrichtung zum Bestimmen von Flüssigkeitsparametern, insbesondere für die Blutgasanalyse. Nach der Erfindung besteht der Behälter aus einem elastischen, gasdichten Material und ist mit einem selbstdichtenden Verbindungselement versehen, das mit einem an der Vorrichtung angeordneten Anschlußdorn in Form einer Hohlnadel zusammenwirkt und diesen immer fest umschließt, so daß zwischen dem Verbindungspfropfen und der Nadel weder Flüssigkeit noch Gas austreten kann. Die Vorrichtung zum Bestimmen der Flüssigkeitsparameter hat mindestens eine Aufnahmeschublade, in die der Flüssigkeitsbehälter eingelegt werden kann, woraufhin die Schublade zusammen mit dem darin liegenden Beutel in Richtung auf die Hohlnadel eingeschoben wird und so automatisch beim Einschieben der Schublade der Anschluß zwischen Beutel und Vorrichtung bewirkt wird. Die Vorrichtung ist bevorzugt mit mindestens einer Sauerstoff-Meßelektrode versehen, die auf dem Clark'schen Elektrodensystem basiert und eine Anode aus Wolfram oder Molybdän oder einem anderen Element der Gruppe VI B des Periodensystems der chemischen Elemente hat. Die Vorrichtung hat ferner vorzugsweise mindestens zwei Meßstrecken mit Meßelektroden zum Bestimmen des Säure-Basegleichgewichts der untersuchten Flüssigkeit, welche Meßelektroden in den jeweiligen Meßstrecken parallelgeschaltet sind und alle an eine gemeinsame Auswerteelektronik angeschlossen sind.

## Beschreibung

Die Erfindung betrifft eine Vorrichtung zum Bestimmen von Flüssigkeitsparametern, insbesondere für die Blutgasanalyse, bei der eine Flüssigkeitsprobe in einer Meßvorrichtung mit mindestens einer Meßelektrode vermessen wird und die Meßvorrichtung zwischen dem Messen zweier Flüssigkeitsproben mit mindestens einer Waschflüssigkeit und mindestens einer Kalibrierflüssigkeit gespült wird, sowie einen Behälter zur Aufnahme der Kalibrier- und/oder Waschflüssigkeit. Die Erfindung ist weiter auf eine Meßelektrode zum Bestimmen von Flüssigkeitsparametern auf der Basis des Clark'schen Elektrodensystems, insbesondere zur Verwendung in der genannten Vorrichtung und auf ein Verfahren zum Bestimmen von Flüssigkeitsparametern gerichtet, insbesondere zum Bestimmen von Blutgaswerten, bei dem nacheinander Proben der zu untersuchenden Flüssigkeit, mindestens eine Kalibrierflüssigkeit und eine Betriebsflüssigkeit durch eine Meßvorrichtung geleitet werden, wobei die chemischen und/oder physikalischen Eigenschaften der zu untersuchenden Flüssigkeit und der Kalibrierflüssigkeit mit Hilfe von Meßelektroden und auf Grundlage der erhaltenen Meßergebnisse die Flüssigkeitsparameter bestimmt werden.

Die Erfindung dient insbesondere zum Bestimmen der Gasparameter von Blut und blutähnlicher Stoffe wie beispielsweise dem pH-Wert, dem Sauerstoff- und Kohlendioxydpartialdruck (pO₂ und pCO₂) sowie der Ionenkonzentrationen von Natrium, Kalium und Chlor (Ma+, K+ und Cl-).

Aus der US-PS 3 874 850 ist ein Verfahren zur Analyse von Blut bekannt, bei dem die Blutprobe durch eine mit Meßvorrichtungen versehene, hydraulische Leitung geleitet wird und Kennwerte wie beispielsweise der PH-Wert und die Gas-Partialdrücke pCO₂ und pO₂ bestimmt werden, und bei dem nach Bestimmung dieser Werte die Blutprobe aus der Leitung wieder entfernt wird. Vor Zufuhr einer neuen Blutprobe in die Leitung wird diese mit einer Waschflüssigkeit gespült und anschließend mit mindestens zwei Kalibrierflüssigkeiten mit verschiedenen, bekannten pH-, pCO₂- und pO₂- Werten kalibriert, die in festliegenden Zeitabständen voneinander und in bestimmter Menge durch die Leitung hindurchfließen und dabei die oben genannten Werte bekannter Größe mit Hilfe der Meßvorrichtungen gemessen werden. Bei diesem Verfahren werden die bekannten und gemessenen Musterwerte der Kalibrierflüssigkeiten und die ermittelten Parameter des untersuchten Blutes gespeichert und weiterverarbeitet, um Blutgasanalyseergebnisse zu erhalten, die leichter interpretierbar sind.

Aus der genannten Druckschrift ist auch ein automatischer Blutprobenanalysator bekannt, der mit einer hydraulischen Leitung mit einem Einlaßabschnitt zum Einführen einer Blutprobe versehen ist und der einen Meßabschnitt mit Meßvorrichtungen zum Bestimmen mindestens eines Blutprobeparameters aufweist, beispielsweise Elektroden zum Ermitteln des pH-Werts, des Kohlendioxydpartialdrucks pCO₂ oder des Sauerstoffpartialdrucks pO₂. Der Analysator hat ferner an den Einlaßabschnitt angeschlossene Behälter für die Waschflüssigkeit und die Kalibrierflüssigkeit und ist mit einer Pumpvorrichtung versehen, mit deren Hilfe eine turbulente Durchströmung in der Leitung erzeugt wird und die verschiedenen Flüssigkeiten aus dem Meßabschnitt der Leitung entfernt werden. Außerdem ist der Analysator mit einer Steuerungseinrichtung mit einem Rechner versehen, mit dessen Hilfe die Pumpvorrichtung gesteuert wird und womit die Temperatur in den Aufbewahrungsbehältern für die Flüssigkeiten und in dem Meßabschnitt der Leitung konstant gehalten wird. Ferner dient der Computer dazu, die von den Meßvorrichtungen gemessenen Musterwerte und die gemessenen Blutparameter in Digitalwerte umzuwandeln.

Aus der EP-PS 0012 031 ist ein Verfahren zum Messen von chemischen Flüssigkeitsparametern bekannt, z.B. von Blutwerten, bei dem eine bestimmte Menge der zu untersuchenden Flüssigkeit in einer Meßkammer eingebracht und in Kontakt mit einer in eine Elektrolytlösung eingetauchten Meßelektrode gebracht wird. Das elektrische Potential zwischen der Meßelektrode und einer Bezugselektrode wird bestimmt. Als Meßergebnis erhält man einen chemischen Wert der Flüssigkeit, die sich in der Meßkammer befindet.

Aus dieser Druckschrift ist auch eine Meßvorrichtung zur chemischen Messung von Flüssigkeitsparametern bekannt, die ein Gehäuse mit einer oberen und unteren Wand aufweist, in dem ein längsverlaufender Kanal ausgebildet ist. Diese Vorrichtung hat eine neben der Längsachse des Kanals angeordnete Meßelektrode mit einem in den Kanal hineinragenden Endstück und eine zur Meßelektrode versetzt angeordnete Bezugselektrode. Außerdem ist in dieser Vorrichtung die Meßkammer angeordnet, die eine Meßbrücke zwischen der Bezugs- und der Meßelektrode bildet, sowie die Vorrichtung für die Zufuhr des Elektrolytes in die Kammer und damit zum Benetzen der Elektrode mit den Elektrolyten, sowie ein Kanal, der die untersuchte Flüssigkeit aus dem Endstück der Meßelektrode zuleitet, um von dieser das Elektrolyt zu verdrängen, wobei das zwischen den Elektroden gemessene Potential einen Parameter der chemisch untersuchten Flüssigkeit darstellt.

Aus der US-PS 3 884 640 ist eine Meßvorrichtung zum Bestimmen von zumindest zwei der folgenden Parameter bekannt: pH-Wert, Partialdruck eines oder mehrerer in der Flüssigkeit gelösten Gase(s), Konzentrationen anorganischer Ionen, Hämoglobin und/oder Temperatur. Die Vorrichtung hat ein Gefäß mit einem Durchströmkanal für eine Flüssigkeit, der mindestens an zwei Stellen mit Verwirbelungseinrichtungen zum Erzeugen einer turbulenten Strömung der Flüssigkeit in einem Bereich versehen ist, in dem die Flüssigkeitsparameter gemessen werden. In der Nähe dieser Verwirbelungseinrichtungen ist mindestens je eine Fühlergruppe im Kanal angeordnet. Das Gefäß ist ferner mit einem Wärmeaustauscher versehen, um eine möglichst gleichbleibende Temperatur der durch den Kanal fließenden Flüssigkeit sicherzustellen. Außerdem ist das Gefäß mit einer Feinanzeigeeinrichtung versehen.

Aus der US-PS 4 871 439 ist ein Analysegerät bekannt, bei dem ein selbstkalibrierendes Elektrodenpaket verwendet wird. Dieses Analysegerät hat eine Meßkammer, die mit unterschiedlichen Elektrodensätzen und einem elektronischen Meß-Steuerungssystem versehen ist.

Zum Messen des Sauerstoff-Partialdrucks pO₂ im Blut sind Elektroden bekannt, die auf dem Clark'schen Elektrodensystem basieren. Die Arbeitsweise solcher Elektroden basiert darauf, daß der Sauerstoff im Blut durch eine Membrane in einen Elektrolyten hineindiffundiert, und seine Konzentration an der Kathodenseite der Elektrode dadurch geringer wird. Gleichzeitig findet an der Anodenseite der Elektrode eine Oxreaktion oder Redox-Reaktion statt. Der gemäß den Clark'schen Elektrodensystem fließende Strom ist dabei proportional zum Partialdruck des Sauerstoffs im Blut. Die Elektroden werden meistens durch Eingabe zweier unterschiedlicher Gasproben mit verschiedenen, bekannten Sauerstoffkonzentrationen kalibriert. Die Kathode einer derartigen Clark'schen Elektrode besteht normalerweise aus Edelmetall, beispielsweise aus Platin oder aus Gold, während die Anode aus Silber besteht oder vorzugsweise mit einer Silberchlorid-Filmbeschichtung versehen ist.

Ein Nachteil einer solchen, auf dem Clark'schen Elektrodensystem basierenden Sauerstoffelektrode liegt darin, daß sich beim Betrieb der Elektrode infolge der Elektrolyse Silber von der Anode auf der Kathode ablagert. Dabei kommt es zu einem zwischen der Anode und der Kathode fließenden Zusatzstrom zusätzlich zu dem infolge der Sauerstoffreduktion an der Kathode fließenden Strom. Dieser Zusatzstrom ist zeitlich veränderlich und bewirkt eine Ungleichmäßigkeit des gesamten, mit der Elektrode gemessenen Stromes und damit Meßfehler bei der Bestimmung des Sauerstoffpatialdrucks p0₂. Außerdem hat das sich an der Kathode anlagernde Silber den Nachteil, daß die Sauerstoffreduktionsgeschwindigkeit sinkt, wodurch die Sauerstoffelektrode eine nicht lineare Charakteristik erhält. Beide nachteiligen Wirkungen nehmen im Laufe der Zeit zu und die Kathodenoberfläche muß regelmäßig gereinigt werden, indem man das angelagerte Silber mechanisch entfernt.

Aus der US-PS 448 629 1 ist eine Sauerstoffelektrode bekannt, bei der zwischen der Anode und der Kathode eine Sperrschicht vorgesehen ist, die Silberionen nicht durchläßt, wodurch eine Ablagerung von Silber an der Kathode verhindert wird.

Aufgabe der Erfindung ist es, eine Vorrichtung und ein Verfahren sowie eine Meßelektrode zum Bestimmen von Flüssigkeitsparametern anzugeben, womit eine besonders einfache und genaue Bestimmung der gesuchten Flüssigkeitsparameter, beispielsweise der Blutgaswerte möglich ist. Ferner soll mit der Erfindung ein besonders vorteilhafter Behälter für die Aufnahme von Kalibrierflüssigkeiten für die Vorrichtung geschaffen werden. Diese Aufgabe wird durch die in den unabhängigen Ansprüchen angegebenen Merkmale gelöst.

Mit der Erfindung wird eine Vorrichtung zum Bestimmen von Flüssigkeitsparametern, insbesondere für die Blutgasanalyse geschaffen, bei der eine Flüssigkeitsprobe in einer Meßvorrichtung mit mindestens einer Meßelektrode vermessen wird und die Meßvorrichtung zwischen dem Messen zweier Flüssigkeitsproben mit mindestens einer Waschflüssigkeit und mindestens einer Kalibrierflüssigkeit gespült wird, wobei wenigstens die Kalibrierflüssigkeit in einem auswechselbaren, gasdichten Behälter angeordnet ist, der mittels eines selbstdichtenden, elastischen Verbindungsstücks mit der Vorrichtung verbindbar ist. Der bzw. die Behälter für die Kalibrierflüssigkeit(en) sind zweckmäßig elastische, gasdichte Beutel und die Vorrichtung ist vorzugsweise mit mindestens einer Aufnahmeeinrichtung für den bzw. die Beutel versehen, die einen oder mehrere mit dem an den Beuteln angeordneten Verbindungsstücken zusammenwirkende(n) Anschlußdorn(e) aufweist, um die Beutel an der Vorrichtung anzuschließen.

Die Ausgestaltung nach der Erfindung hat den Vorteil, daß die Kalibrierflüssigkeit mit dem darin gelösten Kalibriergas unter Luftabschluß in den Behälter eingefüllt, in diesem aufbewahrt und aus diesem wieder entnommen werden kann, so daß die Gaskonzentration in der Flüssigkeit immer konstant ist.

Die Aufnahmeeinrichtung ist zweckmäßig mit einer ein- und ausschiebbaren Schublade zur Aufnahme des Behälters bzw. Beutels versehen, wodurch der Anschluß des Beutels an die Vorrichtung besonders einfach ist. Die Bedienperson muß lediglich die Schublade an der Vorrichtung herausziehen, den Beutel mit seinem Verbindungsstück in Einschubrichtung nach vorne einlegen und dann die Schublade zusammen mit dem Beutel wieder einschieben, wobei im letzten Teil des Einschubweges das Verbindungsstück erst in Berührung mit dem an der Aufnahmeeinrichtung angeordneten Anschlußdorn, vorzugsweise eine Hohlnadel, gelangt und beim weiteren Einschieben die Hohlnadel durch das selbstdichtende Verbindungsstück hindurch ins Innere des Beutels gestochen wird. Das Verbindungsstück ist dabei vorzugsweise mit einem Verschlußpfropfen aus elastischem Gummi versehen, der sich beim Durchstecken der Hohlnadel dicht an deren Außenseite anlegt und somit ein Austreten von Flüssigkeit und von in der Flüssigkeit gelöstem Gas zwischen der Nadel und dem Verbindungsstück sicher verhindert.

Die Vorrichtung zum Bestimmen der Flüssigkeitsparameter weist eine Steuereinheit mit Monitor sowie ein Analysator auf, der eine Meßkammer mit Meßelektroden enthält, an die die Kalibrier- und Waschflüssigkeitsbehälter angeschlossen sind und zu denen auch die zu untersuchende Flüssigkeitsprobe geleitet wird. Der Analysator hat ferner Steuer- und Ausführungselemente, um den Betrieb der Vorrichtung zu steuern und ist mit einer Thermostateinrichtung versehen, um eine gleichbleibende Temperatur an der Meßvorrichtung zu erhalten. Außerdem ist auch eine Recheneinheit vorgesehen, mit deren Hilfe die gemessenen Werte in Werte für die Flüssigkeitsparameter umgerechnet werden. Nach der Erfindung sind in der Vorrichtung die Behälter für die Kalibrierflüssigkeiten auswechselbar angeordnet. In vorteilhafter Ausgestaltung der Erfindung ist mindestens eine der Meßelektroden eine auf dem Clark'schen Elektrodensystem basierende Sauerstoffelektrode, die eine Anode hat, die aus einem Element der Gruppe VI B des Periodensystems der chemischen Elemente besteht, vorzugsweise aus Wolfram oder Molybdän.

In bevorzugter Ausgestaltung der Erfindung weist die Vorrichtung mindesten zwei Meßstrecken in der Meßkammer auf, denen die Meßelektroden zum Bestimmen des Säure-Basegleichgewichts zugeordnet sind, wobei die Meßelektroden in den Meßstrecken parallelgeschaltet und an gemeinsame, elektronische Systeme angeschlossen sind. Die Meßstrecken können unabhängig voneinander kalibriert und die Untersuchung der Blutprobe wahlweise in der einen oder der anderen Meßstrecke untersucht werden, und es ist auch denkbar, daß die eine Meßstrecke mit ihren Elektroden mit der Waschflüssigkeit gereinigt und der bzw. den Kalibrierflüssigkeit(en) neu kalibriert wird, während in der anderen Meßstrecke eine Flüssigkeitsprobe untersucht wird.

Mit der Erfindung wird ein Verfahren zum Bestimmen von Flüssigkeitsparametern, insbesondere zum Bestimmen von Blutgaswerten geschaffen, bei dem nacheinander Proben der zu untersuchenden Flüssigkeit, mindestens eine Kalibrierflüssigkeit und eine Betriebsflüssigkeit durch eine Meßvorrichtung geleitet werden und die chemischen und/oder physikalischen Eigenschaften der zu untersuchenden Flüssigkeit und Kalibrierflüssigkeit(en) mit Hilfe von Meßelektroden gemessen und auf Grundlage der erhaltenen Meßergebnisse die Flüssigkeitsparameter bestimmt werden. Erfindungsgemäß werden dabei als Kalibrierflüssigkeiten für die Meßelektroden in der Meßvorrichtung gasgesättigte Wasserstandardlösungen und Luftsauerstoff verwendet, die im Wechsel mit der zu untersuchenden Flüssigkeitsprobe und der Betriebsflüssigkeit durch wenigstens zwei Meßstrecken in der Meßvorrichtung geleitet werden, wobei in jeder Meßstrecke wenigstens eine Meßelektrode zur Bestimmung des Säure-Base-Gleichgewichts angeordnet ist und wobei die Meßergebnisse eines jeden Meßpfades anschließend in einer gemeinsamen Auswerteelektronik ausgewertet werden.

Ein besonderer Vorteil der Erfindung besteht darin, daß die Kalibriergase oder Normierungsgase, insbesondere Kohlendioxyd, in den in auswechselbaren Behältern befindlichen Kalibrierflüssigkeiten gelöst vorhanden sind, so daß die ganze Vorrichtung nicht an eine zusätzliche Gasversorgung, beispielsweise eine oder mehrere Gasflaschen, angeschlossen werden muß.

Durch die Anordnung von mindestens zwei Meßstrecken in der Meßkammer ist es möglich, schnell von der einen Meßstrecke zur anderen Meßstrecke umzuschalten, beispielsweise wenn dies im Falle einer Störung durch nicht betriebssicher arbeitende Elektroden od.dgl. erforderlich ist. Die Vorrichtung ist dadurch besonders zuverlässig und erlaubt es, die Untersuchungen besonders schnell durchzuführen. Dadurch, daß beide Meßstrecken und die darin angeordneten Elektroden an eine gemeinsame Steuerung angeschlossen sind, die vorzugsweise auch zur Steuerung der hydraulischen Bauteile der Vorrichtung wie Pumpen od.dgl. verwendet wird, kommt die Vorrichtung mit vergleichsweise wenigen Bauelementen aus.

Die erfindungsgemäße Sauerstoffelektrode, die bevorzugt in der Vorrichtung zum Bestimmen von Flüssigkeitsparametern verwendet wird, hat den Vorteil, daß zwischen ihrer Anode und ihrer Kathode kein infolge der Reduktion von Anodenmaterial auf der Kathode entstehender Zusatzstrom fließt und die Kathode auch nicht durch Anodenmaterial verschmutzt wird. Dadurch ist es möglich, die Meßgenauigkeit beim Bestimmen der Sauerstoffparameter durch die Elektrode zu vergrößern und es ist nicht erforderlich, die Kathode regelmäßig von Verunreinigungen zu befreien.

Weitere Merkmale und Vorteile der Erfindung ergeben sich aus der nachfolgenden Beschreibung und den Zeichnungen, worin eine bevorzugte Ausführungsform der Erfindung anhand eines Beispiels näher erläutert ist. Es zeigt:
- Fig. 1: eine schematische Darstellung einer Vorrichtung zum Bestimmen von Flüssigkeitsparametern nach der Erfindung;
- Fig. 2: eine detaillierte, schematische Darstellung des Analysators der Vorrichtung nach Fig. 1;
- Fig. 3: ein Prozeß-Fließbild des gashydraulischen Systems des Analysators nach Fig. 2 mit zwei separaten Meßstrecken;
- Fig. 4: einen Behälter für eine Kalbrierflüssigkeit zur Verwendung in der Vorrichtung nach Fig. 1, in einer Draufsicht.

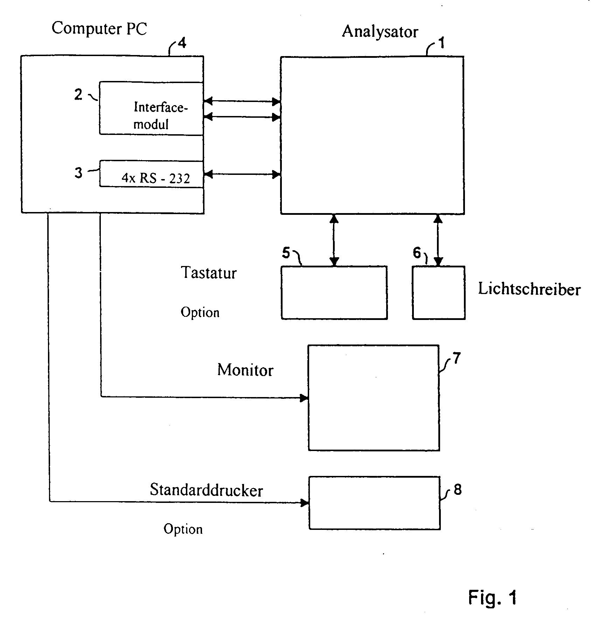

Fig. 1 zeigt in schematischer Darstellung eine Vorrichtung zum Bestimmen von Flüssigkeitsparametern, insbesondere für die Blutgasanalyse, mit einem Analysator 1, der über eine Analog-/Digitalschnittstelle 2 und eine Zusatzschnittstelle 3 mit einer Steuereinheit 4 verbunden ist. Am Analysator 1 ist eine Tastatur 5 und ein Lichtschreiber 6 angeschlossen, während die Steuereinheit 4 mit einem Monitor 7 und einem Drucker 8 verbunden ist. Die Steuereinheit 4 hat ferner einen Arbeitsspeicher, eine Festplatte und ein Diskettenlaufwerk, die in der Zeichnung nicht dargestellt sind, und bildet zusammen mit dem Monitor einen Computer. Der Computer muß nicht unmittelbar an der Meßstelle angeordnet sein und ist mit einem Kabel mit dem Analysator 1 verbunden. Über den Monitor kann der Benutzer die mit der Vorrichtung durchgeführten Untersuchungen unmittelbar visuell verfolgen, wobei der Monitor hierzu zweckmäßig in der Nähe des Analysators 1 angeordnet und direkt an die Graphikkarte im Computer angeschlossen ist.

Die Tastatur 5 kann bei Bedarf verwendet werden und ist an eine Buchse an der Vorderwand des Analysators 1 angeschlossen. Sie dient zur Eingabe von alphanumerischen Daten und zum Testen der Vorrichtung. Die Analog-/Digitalschnittstelle 2 hat Digital- und Analog-Eingabe-und Ausgabepfade, die in Form einer Erweiterungskassette oder -steckkarte im Computer angeordnet sind, wobei der Rechner auf Grundlage eines 16-Bit-Busses arbeitet. Die Steckkarte ist mit dem Rechner über einen Eingang-/Ausgangs-Erweiterungsbus verbunden und arbeitet mit diesem auf der Grundlage von Programminformationen des Prozessors und des Arbeitsspeichers zusammen. Die Steckkarte steht über Meß- und Digitalkabel mit dem Analysator 1 in Verbindung. Die Meßkabel sind abgeschirmte Kabel mit jeweils einer eigenen Abschirmung für jeden Kanal. Die Zusatzschnittstelle 3 dient zur Kommunikation zwischen dem Computer und Zusatzgeräten oder mit einem ggf. übergeordneten Rechner. Der Lichtschreiber 6 dient als Lesegerät, mit dessen Hilfe Streifencodes (bar codes) abgetastet und gelesen werden können, wie sie zur Kennzeichnung von Kalibrierflüssigkeiten, von Blutproben der Patienten, von Identifizierungskarten der Patienten oder der Bedienperson der Vorrichtung verwendet werden und die beispielsweise auf den entsprechenden Behältern angebracht sind. Der Lichtschreiber 6 ist an einer Buchse in einer Wand des Analysators 1 angeschlossen und kommuniziert mit dem Computer über eine Abstimmschaltung, die sich im Analysator 1 oder im Lichtschreiber 6 selbst befindet. Es ist auch möglich, anstelle des Lichtschreibers 6 einen Streifencodeleser mit einer CCD-Ablesematrix zu verwenden. Der Drucker 8 ist ein gebräuchliches Peripheriegerät, das über eine Centronix-Schnittstelle mit dem Rechner verbunden ist. Er dient zum Drucken von Texten, Meßergebnissen usw.

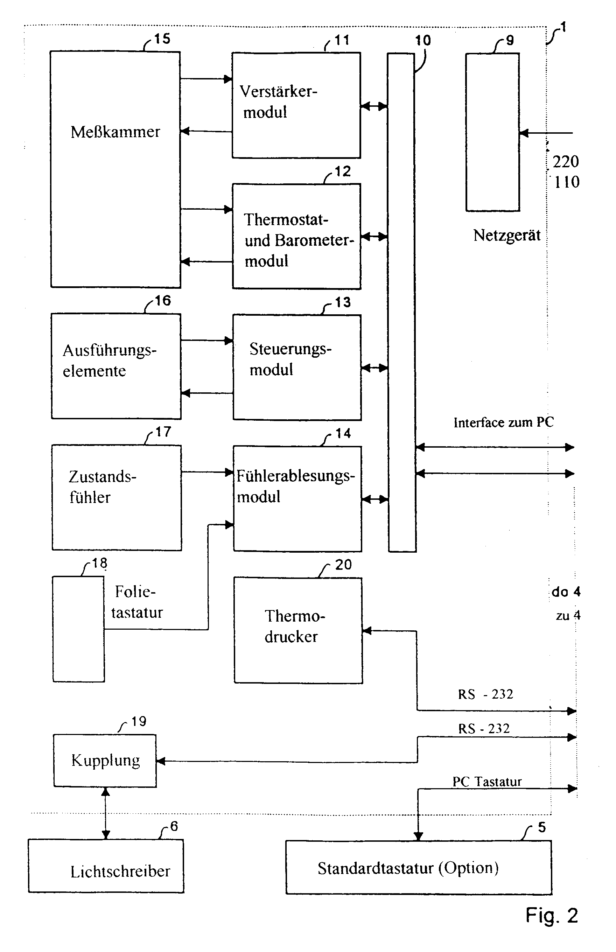

Der Analysator 1 ist in Fig. 2 näher dargestellt. Er ist mit einem Netzgerät 9 versehen, das die einzelnen Bauteile des Analysators mit der erforderlichen Energie versorgt. Der Analysator hat weiter mindestens ein Verstärkermodul 11, eine Thermostat- und Barometereinrichtung 12, ein Steuerungsmodul 13 und ein Fühlerablesemodul 14, die alle an einen gemeinsamen Bus 10 angeschlossen sind. Das Verstärkermodul 11 und die Thermostat- und Barometereinrichtung 12 sind einer Meßkammer 15 zugeordnet und mit dieser verbunden. Am Steuerungsmodul 13 sind Ausführungselemente 16 und am Fühlerablesemodul 14 Zustandfühler 17 und ein Bedienfeld mit einer Tastatur 18 angeschlossen. Zum Analysator 1 gehört ferner eine Anschlußkupplung 19 zum Anschluß des Lichtschreibers 6 und ein Minidrucker 20, der ein Themodrucker sein kann. Die Kupplung 19, der Minidrucker 20, der gemeinsame Bus 10 sowie die Tastatur 5 sind an die Steuereinheit 4 des Computers angeschlossen.

Das Verstärkermoul 11 weist mehrere analoge Vorverstärker auf, die in der Zeichnung nicht dargestellt sind und den Meßelektroden zugeordnet sind, die sich in der Meßkammer 15 befinden. Die Meßelektroden dienen zum Bestimmen des pH-Werts, der Ionenkonzentration, des Sauerstoffpartialdrucks und der Partialdrücke von anderen Gasen in der zu untersuchenden Probe und sollen demgemäß im folgenden kurz pH-Elektrode, Ionenselektivelektrode, pO₂-Elektrode bzw. pCO₂-Elektrode bezeichnet werden. Die Vorverstärker bilden Impedanzwandler für die von den pH- und Ionenselektivelektroden übermittelten Signale und dienen der Verstärkung des Signales von der pH-Elektrode, eines Strompotentialwandlers und des Signales der pO₂-Elektrode. Das Verstärkermodul 11 ist ferner mit einem Analogschlüsselsatz versehen, der in der Zeichnung nicht dargestellt ist und mit dessen Hilfe Selbsttests der einzelnen Verstärker (Null und Kalibrierung) durchgeführt werden können. Die einzelnen Vorverstärker der Verstärkergruppe haben Low-Pass-Filter, die die Signale für den Analog-/Digitalwandler vergleichmäßigen oder standardisieren.

Erfindungsgemäß werden mit der Vorrichtung Sauerstoff- oder pO₂-Elektroden verwendet, die auf dem Clark'schen Elektrodensystem basieren und die eine Anode enthalten, die aus einem Element der Gruppe VI B der Periodensystems der chemischen Elemente, vorzugsweise aus Wolfram oder Molybdän besteht.

Die Thermostat- und Barometereinrichtung 12 besteht im wesentlichen aus zwei unabhängigen Meß- und Schaltvorrichtungen, wovon die eine ein Luftdruckmeßsystem mit einer Meßbrücke und einem Signalverstärker ist, dessen Ausgangssignal einem Analog-Digitalwandler und von dort einer Anzeigevorrichtung zugeleitet wird, an der der gemessene Luftdruck ablesbar ist. Die andere Vorrichtung ist ein elektronischer Thermostat mit einem Temperaturfühler in der Meßkammer 15 und mit Heizelementen, die in Abhängigkeit von der von dem Temperaturfühler gemessenen Temperatur und einer einstellbaren Solltemperatur geregelt werden.

Der Verstärker 11 und die Thermostat- und Barometereinrichtung 12 sind der Meßkammer 15 zugeordnet und mit dieser verbunden, wobei die Meßkammer 15 einen vorzugsweise isolierten, auf gleichbleibender Temperatur gehaltenen Elektrodenblock aus Metall oder Kunststoff bildet. Der Elektrodenblock weist dabei mehrere in das hydraulische System bzw. die Flüssigkeits- und gasführenden Leitungen des Analysators eingeschaltete Elektroden auf, deren Ausgänge direkt an das Verstärkermodul angeschlossen sind. Die Temperatur des Elektrodenblockes wird von dem Thermostat- und Barometermodul 12 konstantgehalten. Hierzu ist der Elektrodenblock mit Heizelementen und mindestens einem Temperaturfühler versehen, die in der Zeichnung nicht dargestellt sind, wobei vorzugsweise drei Temperaturfühler vorgesehen sind, wovon einer zur Temperaturstabilisierung, der zweite zum Überwachen dieser Isothermsteuerung und der dritte zum Bestimmen der Temperatur in der untersuchten Probe und der Kalibrierflüssigkeiten dient.

Das Steuerungsmodul 13 weist mehrere, computergesteuerte, bistabile Leitungsverstärker auf, die zur direkten Steuerung von Ausführungselementen 16 wie beispielsweise Ventilen, Motoren und Pumpen dienen, die Bestandteil des hydraulischen und mechanischen Systems des Analysators 1 sind. Am Steuerungsmodul 13 sind Anzeigevorrichtungen mit Spiegelablesung vorgesehen, die die jeweiligen Betriebszustände der mit dem Steuerungsmodul 13 gesteuerten Aggregate anzeigen und mit deren Hilfe auch Fehler oder Ausfälle eines oder mehrerer Aggregate festgestellt werden können, die sich in von einem Normalwert abweichenden Werten an der jeweiligen Anzeigevorrichtung äußern. Solche Defekte können beispielsweise ein Kurzschluß oder ein Durchbrennen eines Motors oder eines anderen Aggregates sein.

Das Fühlerablesemodul 14 enthält bistabile Systeme zum Ablesen des Zustandes der Folientastatur 18 und des Zustandes oder Ausgangswertes verschiedener Fühler 17, die in der hydraulischen Leitungsanordnung oder der Mechanik des Analysators 1 vorgesehen sind, beispielsweise Fühlstifte, Endfühler oder Fühler, mit denen festgestellt wird, ob eine Flüssigkeit in der hydraulischen Leitungsanordnung vorhanden ist. Mit Hilfe der Folientastatur 18 an der Vorderwand des Analysators wird die Durchführung der Messung gesteuert und können numerische Daten eingegeben werden. Der Minidrucker 20 des Analysators dient zum Ausdrucken der Meß-, Kalibrierungs- und Testergebnisse.

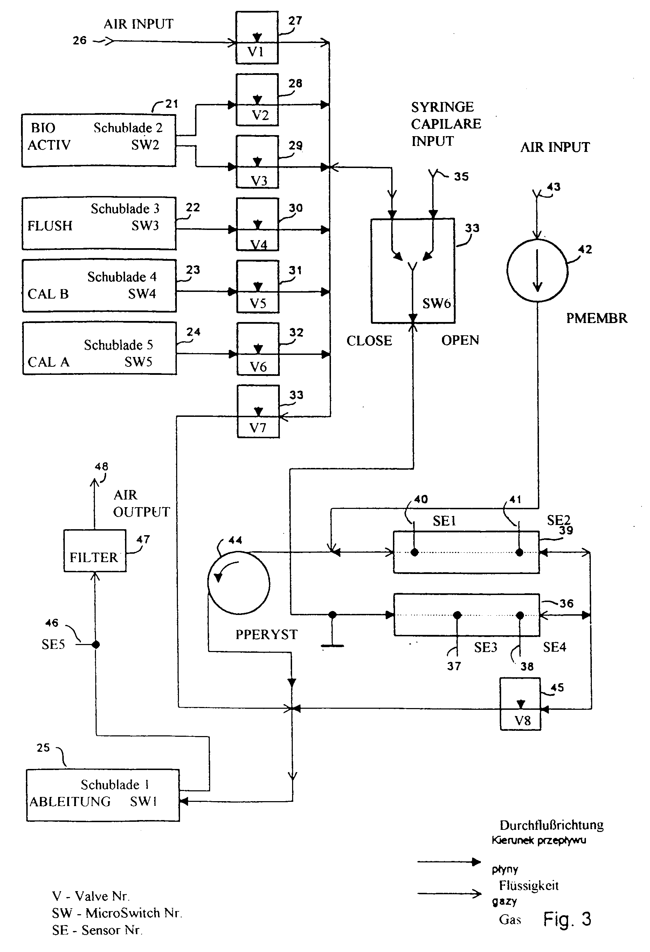

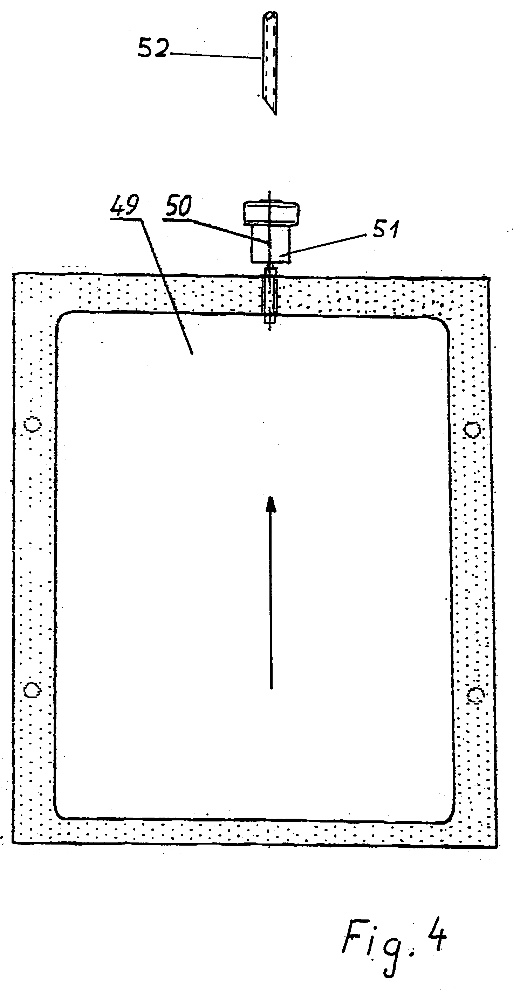

In Fig. 3 ist das gashydraulische Leitungssystem des Analysators 1 schematisch dargestellt. Hierzu gehören mehrere Aufnahmebehälter mit aus- und einschiebbaren Schubladen 21, 22, 23 und 24 zur Aufnahme von auswechselbaren Beuteln, die mit gasgesättigten Kalbrierflüssigkeiten und Betriebsflüssigkeiten gefüllt sind. Die jeweiligen Beutel können dadurch gewechselt werden, daß die zugehörige Schublade herausgezogen wird, wonach der alte, leere Beutel entnommen und ein neuer, die jeweilige Flüssigkeit enthaltender Beutel, in die leere Schublade eingelegt wird. Nach Ablesen des auf dem neuen Beutel angebrachten Streifencodes mit Hilfe des Lichtschreibers 6 wird dann die Schublade mit dem darin befindlichen, neuen, vollen Beutel wieder eingeschoben. Dabei ist die Anordnung so getroffen, daß ein im Aufnahmebehälter angeordneter Anschlußdorn, vorzugsweise eine Hohlnadel, mit ihrer Spitze in Kontakt mit einem am Beutel 49 (Fig. 4) angeordneten Verbindungsstück 50 aus weichelastischem Gummi gelangt und durch dieses Gummi hindurchgestochen wird, wenn die Schublade mit dem darin liegenden Beutel in ihre Normal- oder Betriebsposition geschoben wird. Dabei liegt der Verschlußpfropfen aus selbstklemmendem Gummi dicht an der Außenseite der Kanüle an, so daß zwischen dieser und dem Verschlußpfropfen keine Flüssigkeit und auch kein Gas austreten kann. Derartige Verschlußpropfen werden häufig für Verschlüsse von Flaschen für flüssige Medikamente verwendet, die man mit Hilfe einer Spritze aus der Flasche entnehmen kann.

Ein derartiger Beutel für die Kalibrierflüssigkeiten ist in Fig. 4 dargestellt. Der Beutel besteht aus einem weichelastischen Material, beispielsweise aus Kunststoff und ist an seiner einen Schmalseite mit dem aus Gummi bestehenden Verschlußpfropfen 50 versehen. Dieser hat einen etwa zylindrischen Haltebereich 51, mit dem er an einer in der Schublade angeordneten Halteklammer arretiert werden kann, wodurch immer eine korrekte Ausrichtung des Verschlußpfropfens auf die in der Aufnahme angeordnete Hohlnadel 52 beim Einschieben der Schublade in Pfeilrichtung sichergestellt ist.

Neben den Schubladen 21 bis 24 zur Aufnahme der Kalibrier- und Spülflüssigkeiten ist noch eine Schublade 25 an der Vorrichtung vorgesehen, die einen Abfallbeutel zur Aufnahme von Gebrauchtflüssigkeit, d.h. von bereits benutzter Kalibrierflüssigkeit, Waschflüssigkeit und Blut aufweist. Die Ausgestaltung dieser Schublade und der von dieser aufgenommenen Abfallbeutel ist im wesentlichen dieselbe wie oben beschrieben, mit dem Unterschied, daß in diese Schublade ein leerer Beutel eingelegt wird und ein mit Gebrauchtflüssigkeit gefüllter Beutel wieder entnommen wird.

Die Flüssigkeiten, die sich in dem in den Schubladen 21 bis 24 liegenden Beuteln befindet, sowie Luft von einem Lufteinlaßstutzen 26 werden über Ventile 27, 28, 29, 30, 31 und 32 zu einem hydraulischen Schaltventil 33 geführt oder - falls gewünscht oder erforderlich - über ein Ventil 34 direkt in den Abfallbehälter in der Schublade 25 eingeleitet. An einem zweiten Eingang 35 des hydraulischen Schaltventils 33 wird die zu untersuchende Meßprobe eingegeben. Der Ausgang des hydraulischen Schalters 33 ist mit der Meßkammer verbunden, die eine erste Meßstrecke 36 mit einem Säure-Basegleichgewicht-Meßblock mit mehreren Meßelektroden 37, 38 und eine zweite Meßstrecke 39 aufweist, die einen Säure-Basegleichgewichts-Meßblock mit Meßelektroden 40, 41 hat. Ausgangsseitig ist die Meßkammer mit einem zweiten Lufteinlaßstutzen 43 über eine Luftleitung verbunden, in der eine Membranpumpe 42 eingebaut ist. Am Ausgang der Meßkammer befindet sich ferner eine Schlauchpumpe 44 zum Abfördern der die Meßkammer durchlaufenden Flüssigkeiten zu dem Abfallbehälter in der Abfallschublade 25. Der Ausgang der ersten Meßstrecke 36 ist über ein Ventil 45 ebenfalls an den in der Abfallschublade 25 liegenden Beutel über eine hydraulische Leitung angeschlossen. Von dort aus führt eine weitere hydraulische Leitung mit einer zusätzlichen Elektrode 46 zu einem Filter 47 und von dort zu einem Luftauslaßstutzen 48.

Die hydraulischen Aggregate des Analysators, wie beispielsweise die Ventile 27 bis 35, das hydraulische Schaltventil 33 und die Pumpen 42 und 44 in Fig. 3 werden von dem Steuerungsmodul 13 vom Computer aus gesteuert. In dieser Fig. sind auch die Durchflußrichtungen der Flüssigkeiten und Gase angedeutet. Dabei ist festzustellen, daß die verschiedenen Gase (beispielsweise Luft) und Flüssigkeiten während der Kalibrierung der Vorrichtung und der Untersuchung der Blutproben verschiedene Durchflußrichtungen haben können.

Erfindungsgemäß werden zum Messen der Flüssigkeitsparameter der zu untersuchenden Flüssigkeitsprobe der Meßkammer 15 in von der Steuereinheit 4 festgelegten Zeitabständen nacheinander Proben der zu untersuchenden Flüssigkeiten, die Kalibrierungsflüssigkeiten und Betriebsflüssigkeiten oder Waschflüssigkeiten zugeführt. Als Kalibrierflüssigkeiten können gasgesättigte Wasserstandardlösungen verwendet werden, worunter mit Kohlendioxyd, ggf. auch mit Sauerstoff oder anderen Gasen gesättigte Pufferlösungen mit einem bestimmten, bekannten Partialdruck der darin gelösten Gase zu verstehen sind. Der erforderliche Sauerstoff kann auch aus der Luft entnommen werden. Die Flüssigkeiten können wahlweise entweder der ersten Meßstrecke 36 oder der zweiten Meßstrecke 39 zugeführt werden oder auch auf beide Meßstrecken verteilt werden und mit Hilfe der in den Meßstrecken 36, 39 angebrachten Meßelektroden auf ihre physikalischen und chemischen Eigenschaften untersucht werden. Die Meßergebnisse einer jeden Meßstrecke werden dann von der gemeinsamen Auswerteeinrichtung, bei der vorliegenden Ausführungsform dem Computer, ausgewertet.

## Patentansprüche

1. Behälter für Kalibrierflüssigkeiten für eine Vorrichtung zum Bestimmen von Flüssigkeitsparametern, insbesondere für die Blutgasanalyse, der aus einem gasdichten, elastischen Material besteht und mit einem Verbindungselement zum Anschluß an eine Flüssigkeitsleitung versehen ist, **dadurch gekennzeichnet, daß** das Verbindungselement (50) ein selbstdichtender, elastischer Verbindungspfropfen ist, mit dessen Hilfe die Dichtheit der Verbindung zwischen dem Behälter und der Flüssigkeitsleitung der Vorrichtung und die Dichtheit des Behälters während seines Befüllens, seiner Aufbewahrung, seiner Installierung in der Vorrichtung und seiner Verwendung in der Vorrichtung sichergestellt ist, so daß die Kalibrierflüssigkeit im Behälter während der Aufbewahrung,des Installierens und der Entnahme aus dem Behälter eine konstante Sättiungsgaskonzentration hat.

2. Sauerstoffelektrode auf Basis des Clark'schen Elektrodensystems für eine Vorrichtung zum Bestimmen von Flüssigkeitsparametern, insbesondere für die Blutgasanalyse, **gekennzeichnet durch** eine Anode aus einem Werkstoff der Gruppe VI B des Periodensystems der chemischen Elemente.

3. Sauerstoffelektrode nach Anspruch 2, **dadurch gekennzeichnet, daß** die Anode aus Wolfram besteht.

4. Sauerstoffelektrode nach Anspruch 2, **dadurch gekennzeichnet, daß** die Anode aus Molybdän besteht.

5. Vorrichtung zum Bestimmen von Flüssigkeitsparametern, insbesondere für die Blutgasanalyse, mit einem Analysator und einer Steuereinheit mit Monitor, wobei der Analysator eine Meßkammer mit Meßelektroden aufweist, an die mindestens ein Kalibrierflüssigkeitsbehälter und mindestens ein Betriebsflüssigkeitsbehälter angeschlossen sind und denen eine Probe der zu untersuchenden Flüssigkeit zugeleitet wird, sowie mit Steuer- und Ausführungselementen zum Steuern des Betriebs der Vorrichtung und zum Aufrechthalten einer konstanten Temperatur, sowie mit Bauteilen zum Umrechnen der gemessenen Werte in Werte für die Flüssigkeitsparameter, **dadurch gekennzeichnet, daß** der bzw. die Kalibrierflüssigkeitsbehälter als auswechselbare Behälter nach Anspruch 1 ausgebildet sind.

6. Vorrichtung nach Anspruch 5, **dadurch gekennzeichnet, daß** sie Sauerstoffelektroden nach einem der Ansprüche 2 bis 4 aufweist.

7. Vorrichtung nach Anspruch 5 oder 6, **dadurch gekennzeichnet, daß** die Meßkammer mindestens zwei Meßstrecken (36, 39) mit Meßelektroden (37, 38 bzw. 40, 41) zum Bestimmen des Säure-Base-Gleichgewichts der untersuchten Flüssigkeit bzw. der Kalibrierflüssigkeit aufweist, welche Meßelektroden in den Meßstrecken parallel geschaltet und alle an eine gemeinsame Auswerteelektronik (11, 4) angeschlossen sind.

8. Verfahren zum Bestimmen von Flüssigkeitsparametern, insbesondere zum Bestimmen von Blutgaswerten, bei dem nacheinander Proben der zu unersuchenden Flüssigkeit, mindestens eine Kalibrierflüssigkeit und eine Betriebsflüssigkeit durch eine Meßvorrichtung geleitet werden, wobei die chemischen und/oder physikalischen Eigenschaften der zu untersuchenden Flüssigkeit und der Kalibrierflüssigkeit(en) mit Hilfe von Meßelektroden gemessen und auf Grundlage der erhaltenen Meßergebnisse die Flüssigkeitsparameter bestimmt werden, **dadurch gekennzeichnet, daß** als Kalibrierflüssigkeit für die Meßelektroden in der Meßvorrichtung gasgesättigte Wasserstandardlösungen und Luftsauerstoff verwendet werden, die im Wechsel mit der zu messenden Flüssigkeitsprobe und der Betriebsflüssigkeit durch wenigstens zwei Meßstrecken in der Meßvorrichtung geleitet werden, wobei in jeder Meßstrecke wenigstens eine Meßelektrode zur Bestimmung des Säure-Base-Gleichgewichts angeordnet ist und wobei die Meßergebnisse eines jeden Meßpfades anschließend in einer gemeinsamen Auswerteelektronik ausgewertet werden.
